# FASCICULE DE BREVET EUROPEEN

(11) **EP 3 074 064 B1**
(45) Date de publication et mention de la délivrance du brevet: **11.09.2019**
(21) Numéro de dépôt: 14821732.6
(22) Date de dépôt: 21.11.2014
(51) Int. Cl.: A61M 5/20

(54) **AUTOINJECTEUR**
AUTOMATISCHER INJEKTOR
AUTO-INJECTOR

(30) Priorité: 25.11.2013 FR 1361557
(43) Date de publication de la demande: 05.10.2016
(73) Titulaire: APTAR France SAS, 27110 Le Neubourg (FR)
(72) Inventeur: FABIEN, David, 22700 Saint Quay Perros (FR)
(74) Mandataire: CAPRI
(86) Numéro de dépôt international: PCT/FR2014/052997
(87) Numéro de publication internationale: WO 2015/075399

(56) Documents cités:
- EP-A1- 2 468 328
- WO-A1-2009/040602
- WO-A1-2012/000832
- FR-A1- 2 884 722
- FR-A1- 2 990 862
- US-A1- 2013 324 925

## Description

La présente invention concerne un autoinjecteur.

Les autoinjecteurs sont bien connus dans l'état de la technique. Ces dispositifs ont principalement pour but de réaliser une injection automatique du contenu d'une seringue à l'intérieur du corps d'un patient. Divers systèmes existent pour rendre automatique la pénétration de l'aiguille dans le corps du patient ainsi que l'injection du produit fluide contenu dans la seringue. Les autoinjecteurs sont des dispositifs relativement complexes qui doivent répondre à un certain nombre d'exigences de contraintes pour être fiables. La robustesse du dispositif, sa maniabilité, et sa facilité d'utilisation pour l'utilisateur sont également des éléments importants. Par ailleurs, la plupart de ces autoinjecteurs étant à usage unique, le coût de fabrication et d'assemblage est également un facteur dont il faut tenir compte.

Il existe de nombreux autoinjecteurs sur le marché, qui présentent toutefois tous un certain nombre d'inconvénients.

Ainsi, pour éviter un déclenchement intempestif de l'autoinjecteur, par exemple pendant un transport ou pendant le stockage, les dispositifs doivent comporter des moyens de verrouillage fiables. De même, lorsqu'un utilisateur souhaite utiliser l'autoinjecteur et qu'il déverrouille le dispositif, par exemple en retirant le capot, le dispositif ne doit pas se déclencher de manière intempestive mais seulement au moment où l'utilisateur le souhaite réellement, c'est-à-dire au moment où il l'applique contre la partie du corps dans laquelle il veut réaliser l'injection. Or, notamment quand les personnes utilisant l'autoinjecteur sont des personnes âgées ou handicapées, il peut arriver que l'utilisateur laisse tomber le dispositif au moment où il souhaite l'utiliser. Il est souhaitable que dans un tel cas l'autoinjecteur ne se déclenche pas tout seul. Il est donc important de prévoir une serrure de déclenchement fiable. D'un autre côté, il ne faut pas que l'utilisation de l'autoinjecteur devienne trop difficile, ce qui empêcherait des personnes faibles de l'utiliser. Il est donc difficile de trouver le bon compromis entre la sécurité du verrouillage et la facilité d'utilisation et d'actionnement de l'autoinjecteur. C'est un des objectifs de la présente invention de répondre à ce problème.

De plus, afin d'éviter tout risque de blessures après l'utilisation du dispositif, l'autoinjecteur doit comprendre un dispositif de sécurité aiguille qui évite que l'aiguille reste apparente après l'utilisation du dispositif. Ce dispositif de sécurité doit évidemment également être fiable et ne pas se libérer trop facilement. Il doit aussi être fonctionnel même si l'utilisateur actionne mal l'autoinjecteur, par exemple s'il le retire trop tôt de son corps, avant la fin de l'injection.

Les documents WO2012045833, FR2884722, WO9632974 et WO2012000832 décrivent des dispositifs de l'état de la technique.

La présente invention a pour but de fournir un autoinjecteur qui ne reproduit pas les inconvénients susmentionnés, et qui permet de répondre aux différentes exigences et contraintes importantes pour une utilisation sûre et fiable de l'autoinjecteur.

La présente invention a aussi pour but de fournir un autoinjecteur qui soit fiable d'utilisation, qui soit sûr et qui empêche tout risque de blessure, et qui soit simple et peu coûteux à fabriquer et à assembler.

La présente invention a donc pour objet un autoinjecteur comportant un corps adapté à recevoir un réservoir, ledit réservoir contenant du produit fluide et comportant un piston et une aiguille, tel qu'une seringue pré-remplie, ledit autoinjecteur comportant un manchon actionneur comportant une extrémité de contact destinée à venir en contact avec le corps de l'utilisateur, ledit manchon actionneur étant déplaçable par rapport audit corps entre des positions projetées, dans lesquelles ledit manchon actionneur fait au moins partiellement saillie hors dudit corps, et une position d'actionnement, dans laquelle ledit manchon actionneur est axialement déplacé vers l'intérieur dudit corps, ledit manchon actionneur étant dans une première position projetée avant actionnement de l'autoinjecteur et dans une seconde position projetée après actionnement de l'autoinjecteur, l'un parmi ledit manchon actionneur et ledit corps comportant une patte flexible adaptée à se déformer latéralement par rapport audit manchon actionneur et/ou par rapport audit corps lorsque ledit manchon actionneur est déplacé de sa première position projetée vers sa position d'actionnement puis de sa position d'actionnement en retour vers sa seconde position projetée, l'autre parmi ledit manchon actionneur et ledit corps comportant une zone initiale qui coopère avec ladite patte flexible dans ladite première position projetée, une zone intermédiaire qui coopère avec ladite patte flexible dans ladite position d'actionnement, et une zone de réception finale qui coopère avec ladite patte flexible dans ladite seconde position projetée, ladite zone de réception finale étant décalée au moins latéralement de ladite zone initiale, une paroi axiale déformable étant adaptée à se déformer élastiquement pour laisser passer ladite patte flexible de ladite zone initiale vers ladite zone intermédiaire, ladite paroi axiale déformable, dans sa position non déformée, étant ensuite adaptée à guider ladite patte flexible de ladite zone intermédiaire vers ladite zone de réception finale.

Avantageusement, ladite patte flexible est déformée latéralement lorsque ledit manchon actionneur se déplace de sa première position projetée, avant actionnement, vers sa position d'actionnement, et/ou ladite patte flexible est déformée latéralement lorsque ledit manchon actionneur se déplace de sa position d'actionnement vers sa seconde position projetée, en fin d'utilisation.

Avantageusement, ledit manchon actionneur comporte au moins une rainure initiale reliant ladite zone initiale à ladite zone intermédiaire, ladite rainure initiale comportant ladite paroi axiale déformable.

Avantageusement, ladite rainure initiale comporte un épaulement saillant latéralement dans ladite rainure initiale.

Avantageusement, ledit épaulement est disposé en face de ladite paroi axiale déformable.

Avantageusement, ledit épaulement comporte une paroi inclinée tournée en direction de ladite zone initiale, ladite paroi inclinée déformant latéralement ladite patte flexible de telle sorte que ladite patte flexible déforme ladite paroi axiale déformable.

Avantageusement, ladite rainure initiale est sensiblement axiale et est reliée à ladite zone intermédiaire par une seconde rainure inclinée et/ou une troisième rainure axiale.

Avantageusement, ladite zone de réception finale est reliée à ladite zone intermédiaire par une rainure finale, un épaulement axial étant prévu entre ladite zone de réception finale et ladite rainure finale, ladite patte flexible étant adaptée à coulisser dans ladite rainure finale lorsque ledit manchon actionneur revient de sa position d'actionnement vers sa seconde position projetée, ladite patte flexible venant s'encliqueter sous ledit épaulement axial lorsque ledit manchon actionneur atteint sa seconde position projetée, après utilisation, verrouillant ainsi ledit manchon actionneur par rapport audit corps.

Avantageusement, ladite zone de réception finale est décalée axialement par rapport à ladite zone initiale.

Avantageusement, ladite rainure finale est inclinée et comporte ladite paroi axiale déformable.

Avantageusement, ledit encliquetage de ladite patte flexible dans ladite zone de réception finale génère un son, tel qu'un clic.

Avantageusement, ladite déformation élastique de ladite paroi axiale déformable génère un son, tel qu'un clic.

Avantageusement, ladite patte flexible comporte une tête coulissant dans lesdites rainures initiale et finale entre lesdites zones initiale, intermédiaire et de réception finale, ladite tête comportant une paroi d'extrémité frontale au moins partiellement inclinée adaptée à coopérer avec une extrémité inclinée de ladite paroi axiale déformable pour guider ladite tête dans ladite rainure finale lorsque ledit manchon actionneur revient de sa position d'actionnement vers sa seconde position projetée.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
La figure 1 est une vue schématique partiellement découpée d'un autoinjecteur selon un mode de réalisation avantageux,
Les figures 2 à 7 sont des vues schématiques partielles illustrant en détail la serrure lors des différentes séquences d'utilisation de l'autoinjecteur de la figure 1, et
Les figures 8 à 12 sont des vues schématiques partielles illustrant en détail la serrure lors de différentes séquences d'utilisation de l'autoinjecteur selon un second mode de réalisation avantageux.

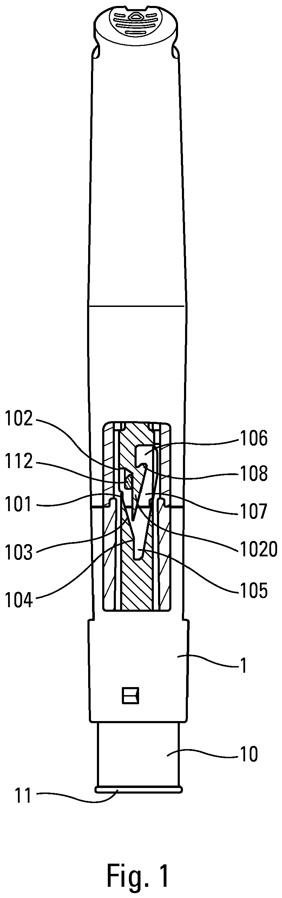

L'autoinjecteur représenté sur la figure 1 comporte un corps 1 dans lequel coulisse axialement un manchon actionneur 10, dont l'extrémité inférieure 11 (dans l'orientation de la figure 1) est destinée à venir en contact avec le corps du patient autour de la zone d'injection. De manière connue, ce corps 1 contient un réservoir contenant le produit à injecter, une aiguille à travers laquelle le produit est distribué, un piston adapté à se déplacer dans ledit réservoir pour réaliser cette distribution, ainsi qu'un système d'injection automatique adapté à coopérer avec ledit piston pour le déplacer. Ces éléments, classiques dans un autoinjecteur, ne sont pas représentés sur les dessins dans des buts de clarté, et ils pourraient être réalisés d'une quelconque manière appropriée, notamment en étant similaires à ceux décrits dans les documents FR 2 990 862, FR 2 990 863, FR 2 990 864, FR 2 990 865, FR 2 990 866, FR 2 990 867, FR 2 990 868, FR 2 990 869 et FR 2 990 870. Le réservoir peut typiquement être une seringue pré-remplie classique.

Avant actionnement, le manchon actionneur 10 est dans une première position projetée dans laquelle il entoure l'aiguille. Lors de l'actionnement, le manchon actionneur 10 coulisse à l'intérieur du corps 1 vers une position d'actionnement, pour exposer l'aiguille et permettre le piquage et l'injection du produit fluide. Après l'injection, le manchon actionneur 10 revient dans une seconde position projetée, dans laquelle il est à nouveau disposé autour de l'aiguille, pour éviter tout risque de blessure avec ladite aiguille. Le manchon actionneur 10 est avantageusement sollicité vers ses positions projetées par un ressort 5, qui peut être de type quelconque.

Le manchon actionneur 10 comporte une rainure initiale 101, avantageusement axiale, qui s'étend depuis une zone initiale 102 vers une zone intermédiaire 105. Ladite rainure initiale 101 comporte une paroi axiale 1020 élastiquement déformable. Avantageusement, une seconde rainure 103, de préférence inclinée, et/ou une troisième rainure 104, avantageusement axiale, relie(nt) ladite rainure initiale 101 à ladite zone intermédiaire 105. Ledit manchon actionneur 10 comporte aussi une zone de réception finale 106 décalée au moins latéralement par rapport à ladite zone initiale 102, et reliée à ladite zone intermédiaire 105 par une rainure finale 107, avantageusement inclinée. Un épaulement axial 108 est prévu entre ladite zone de réception finale 106 et ladite rainure finale 107.

Le corps 1 comporte une patte 110 flexible latéralement, c'est-à-dire qu'elle se déforme dans la direction périphérique du corps. Cette patte flexible 110 comporte avantageusement une tête 112 qui va coopérer avec les rainures et épaulements du manchon actionneur 10, comme cela sera décrit ci-après.

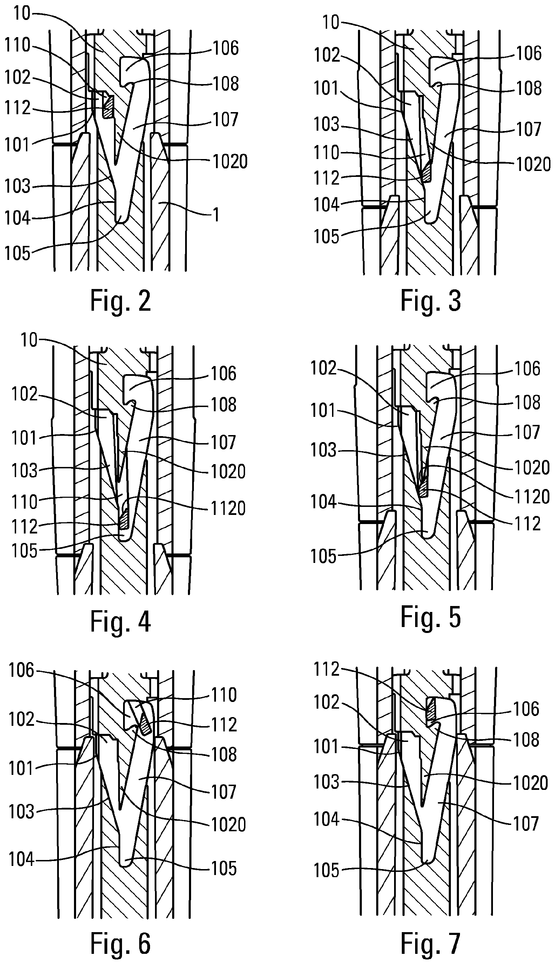

Les figures 2 à 7 illustrent plus particulièrement le fonctionnement de la serrure formée entre le manchon actionneur 10 et le corps 1.

La figure 2 représente la position de départ, c'est-à-dire au moment où l'utilisateur va commencer à utiliser l'autoinjecteur. On voit sur cette figure 2 que la tête 112 de la patte flexible 110 est disposée dans ladite zone initiale 102 de ladite rainure initiale 101. Lorsque le manchon actionneur 10 coulisse vers l'intérieur du corps 1, ladite tête 112 de la patte flexible 110 va coulisser à l'intérieur de ladite rainure initiale 101. Lorsque la tête 112 atteint la position de la figure 3, dans laquelle elle coopère d'un côté avec la seconde rainure 103 et de l'autre côté avec la paroi axiale déformable 1020, elle va déformer latéralement ladite paroi axiale déformable 1020, pour pouvoir continuer sa course d'actionnement. La déformation de la paroi axiale déformable 1020 permet à la tête 112 de la patte flexible 110 de passer dans la zone intermédiaire 105, notamment à travers la troisième rainure axiale 104. Par ailleurs, au moment de la déformation maximale de ladite paroi axiale déformable 1020, celle-ci peut générer un son, tel qu'un clic, pour signaler à l'utilisateur que l'injection va débuter.

Dans la position représentée sur la figure 4, le manchon actionneur 10 a atteint sa position d'actionnement dans laquelle l'aiguille a pénétré dans la zone d'injection du patient jusqu'à la position d'injection et dans laquelle ledit manchon actionneur 10 est apte à actionner le système d'injection. Dans cette position d'actionnement du manchon actionneur 10, la tête 112 de la patte flexible 110 est dans la zone intermédiaire 105. Dans cette position, la paroi axiale déformable 1020 est revenue élastiquement vers sa position non déformée, de sorte que ladite zone intermédiaire 105 est reliée à ladite zone de réception finale 106 par ladite rainure finale inclinée 107.

Lorsque ledit manchon actionneur 10 revient de sa position d'actionnement vers sa seconde position projetée sous l'effet du ressort 5, ladite tête 112 de la patte flexible 110 coulisse dans ladite rainure finale inclinée 107. La figure 5 illustre schématiquement que la patte flexible 110, en particulier la tête 112, ne peut plus revenir dans ladite rainure initiale 101 en raison de la paroi axiale déformable 1020 qui empêche le passage de ladite tête 112. Avantageusement, ladite tête 112 comporte une paroi frontale au moins partiellement inclinée 1120 qui va coopérer avec l'extrémité de ladite paroi axiale déformable 1020 pour guider ladite tête dans la rainure finale inclinée 107, comme visible sur la figure 5. Ainsi, la paroi axiale déformable 1020 fait partie intégrante de la came, formée par la rainure finale 107, qui va guider la patte flexible 110 vers la zone de réception finale 106.

Lorsque ledit manchon actionneur 10 atteint sa seconde position projetée, après utilisation, ladite tête 112 vient s'encliqueter dans ladite zone de réception finale 106 sous ledit épaulement axial 108, verrouillant ainsi ledit manchon actionneur 10 par rapport audit corps 1. A partir de cette position verrouillée, ledit manchon actionneur 10 ne peut plus être déplacé en direction de sa position d'actionnement, de par la butée formée entre la tête 112 de la patte flexible 110 et l'épaulement axial 108. La déformation latérale de la patte flexible 110 lors de l'actionnement, notamment dans la rainure finale inclinée 107 comme illustré sur la figure 6, provoque un encliquetage élastique automatique de la tête 112 sous l'épaulement axial 108 dès que la tête 112 arrive dans ladite zone de réception finale 106, comme illustré sur la figure 7. Le dispositif de sécurité est alors en position finale verrouillée. Ainsi, l'aiguille est totalement protégée après utilisation et l'utilisateur ne peut plus utiliser l'autoinjecteur ou se blesser avec l'aiguille. Cet encliquetage dans la zone de réception finale 106 peut générer un son, tel qu'un clic, pour informer l'utilisateur que la position finale verrouillée est atteinte.

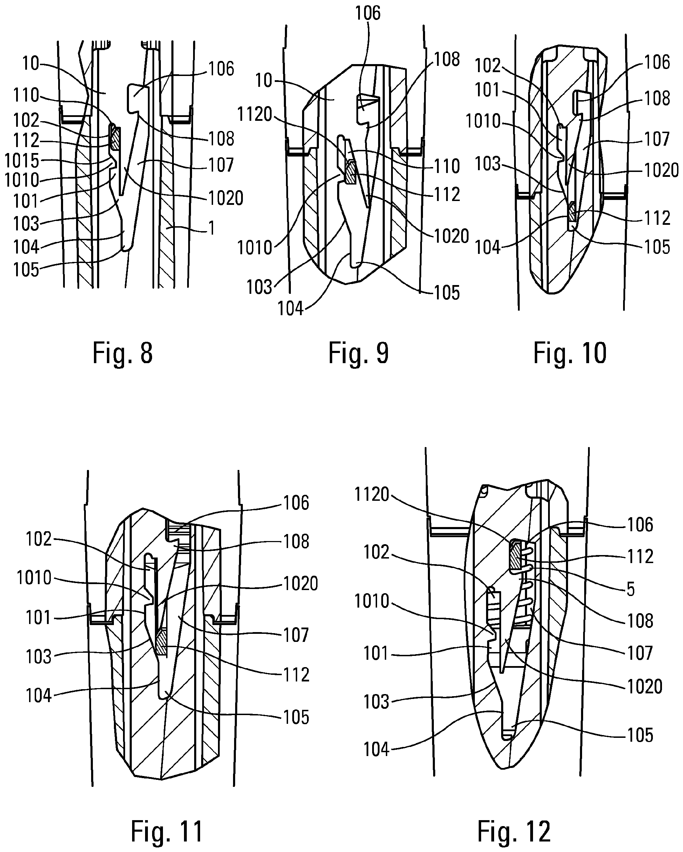

Les figures 8 à 12 illustrent un second mode de réalisation avantageux de l'invention, qui est très similaire au premier mode de réalisation décrit ci-dessus. La seule différence est que la rainure initiale 101 comporte un épaulement 1010 saillant latéralement dans ladite rainure initiale 101, ledit épaulement 1010 comportant de préférence une paroi inclinée 1015 tournée en direction de ladite zone initiale 102. Avantageusement, cet épaulement 1010 est prévu en face de ladite paroi axiale 1020 élastiquement déformable. Lorsque la tête 112 atteint la paroi inclinée 1015 de l'épaulement 1010 dans la rainure initiale 101, ladite paroi inclinée 1015 va coopérer avec ladite tête 112. Cet épaulement 1010 va donc déformer latéralement la patte flexible 110. Ceci va à son tour déformer latéralement la paroi axiale déformable 1020, comme visible sur la figure 9. L'épaulement 1010 forme donc un point dur en début de phase d'actionnement, qui doit être surmonté pour permettre l'actionnement de l'autoinjecteur. Ce point dur combine en particulier la déformation de la patte flexible 110 et la déformation de la paroi axiale déformable 1020, et dans ce second mode de réalisation, la force exercée par l'utilisateur sur le manchon actionneur 10 doit être suffisante pour permettre de réaliser ces deux déformations. Il se produit donc une accumulation d'énergie, qui est libérée au moment où la tête 112 de la patte flexible 110 va passer au-delà de l'épaulement 1010. Ceci garantit que la course d'actionnement sera complète, avec la tête de la patte flexible passant dans la zone intermédiaire 105, notamment à travers la seconde rainure inclinée 103 et la troisième rainure axiale 104.

Les deux variantes de serrure du manchon actionneur décrites ci-dessus sont particulièrement efficaces et fiables, tout en étant robustes et faciles, et donc peu coûteuses, à mouler et à assembler. En particulier, elles ne comportent que deux pièces, le manchon actionneur 10 et le corps 1.

Bien entendu, les formes des rainures, leurs dimensions et leurs inclinaisons peuvent être modifiées en fonction des besoins et des caractéristiques souhaitées pour le dispositif de sécurité aiguille. En particulier, la rainure initiale peut être axiale ou inclinée. Elle peut mener directement à la zone intermédiaire, sans qu'il n'y ait de seconde et troisième rainures. Avec une rainure initiale inclinée, la rainure finale pourrait être axiale ou aussi inclinée. D'autres variantes sont envisageables.

II est à noter que les moyens décrits ci-dessus pourraient être réalisés de manière inversée, c'est-à-dire que le corps 1 pourrait comporter les diverses rainures 101, 103, 104 et 107, les épaulements 1010 et 108 et la paroi axiale déformable 1020, et le manchon actionneur 10 pourrait comporter la patte flexible 110. Dans ce cas, les formes et orientations desdites rainures seraient bien entendu adaptées en conséquence.

Bien que la présente invention ait été décrite en référence à deux modes de réalisation avantageux, il est entendu que l'homme du métier peut y apporter diverses modifications sans sortir du cadre de la présente invention tel que défini par les revendications annexées.

## Revendications

1. Autoinjecteur comportant un corps (1) adapté à recevoir un réservoir, ledit réservoir contenant du produit fluide et comportant un piston et une aiguille, tel qu'une seringue pré-remplie, ledit autoinjecteur comportant un manchon actionneur (10) comportant une extrémité de contact (11) destinée à venir en contact avec le corps de l'utilisateur, ledit manchon actionneur (10) étant déplaçable par rapport audit corps (1) entre des positions projetées, dans lesquelles ledit manchon actionneur (10) fait au moins partiellement saillie hors dudit corps (1), et une position d'actionnement, dans laquelle ledit manchon actionneur (10) est axialement déplacé vers l'intérieur dudit corps (1), ledit manchon actionneur (10) étant dans une première position projetée avant actionnement de l'autoinjecteur et dans une seconde position projetée après actionnement de l'autoinjecteur, l'un parmi ledit manchon actionneur (10) et ledit corps (1) comportant une patte flexible (110) adaptée à se déformer latéralement par rapport audit manchon actionneur (10) et/ou par rapport audit corps (1) lorsque ledit manchon actionneur (10) est déplacé de sa première position projetée vers sa position d'actionnement puis de sa position d'actionnement en retour vers sa seconde position projetée, l'autre parmi ledit manchon actionneur (10) et ledit corps (1) comportant une zone initiale (102) qui coopère avec ladite patte flexible (110) dans ladite première position projetée, une zone intermédiaire (105) qui coopère avec ladite patte flexible (110) dans ladite position d'actionnement, et une zone de réception finale (106) qui coopère avec ladite patte flexible (110) dans ladite seconde position projetée, ladite zone de réception finale (106) étant décalée au moins latéralement de ladite zone initiale (102), **caractérisé en ce qu'**une paroi axiale déformable (1020) est adaptée à se déformer élastiquement pour laisser passer ladite patte flexible (110) de ladite zone initiale (102) vers ladite zone intermédiaire (105), ladite paroi axiale déformable (1020), dans sa position non déformée, étant ensuite adaptée à guider ladite patte flexible de ladite zone intermédiaire (105) vers ladite zone de réception finale (106), ladite patte flexible (110) comportant une tête (112) coulissant dans lesdites rainures initiale et finale (101, 107) entre lesdites zones initiale (102), intermédiaire (105) et de réception finale (106).

2. Autoinjecteur selon la revendication 1, dans lequel ladite patte flexible (110) est déformée latéralement lorsque ledit manchon actionneur (10) se déplace de sa première position projetée, avant actionnement, vers sa position d'actionnement, et/ou ladite patte flexible (110) est déformée latéralement lorsque ledit manchon actionneur (10) se déplace de sa position d'actionnement vers sa seconde position projetée, en fin d'utilisation.

3. Autoinjecteur selon la revendication 2, dans lequel ledit manchon actionneur (10) comporte au moins une rainure initiale (101) reliant ladite zone initiale (102) à ladite zone intermédiaire (105), ladite rainure initiale (101) comportant ladite paroi axiale déformable (1020).

4. Autoinjecteur selon la revendication 3, dans lequel ladite rainure initiale (101) comporte un épaulement (1010) saillant latéralement dans ladite rainure initiale (101).

5. Autoinjecteur selon la revendication 4, dans lequel ledit épaulement (1010) est disposé en face de ladite paroi axiale déformable (1020).

6. Autoinjecteur selon la revendication 4 ou 5, dans lequel ledit épaulement (1010) comporte une paroi inclinée (1015) tournée en direction de ladite zone initiale (102), ladite paroi inclinée (1015) déformant latéralement ladite patte flexible (110) de telle sorte que ladite patte flexible déforme ladite paroi axiale déformable (1020).

7. Autoinjecteur selon l'une quelconque des revendications 3 à 6, dans lequel ladite rainure initiale (101) est sensiblement axiale et est reliée à ladite zone intermédiaire (105) par une seconde rainure (103) inclinée et/ou une troisième rainure (104) axiale.

8. Autoinjecteur selon l'une quelconque des revendications précédentes, dans lequel ladite zone de réception finale (106) est reliée à ladite zone intermédiaire (105) par une rainure finale (107), un épaulement axial (108) étant prévu entre ladite zone de réception finale (106) et ladite rainure finale (107), ladite patte flexible (110) étant adaptée à coulisser dans ladite rainure finale (107) lorsque ledit manchon actionneur (10) revient de sa position d'actionnement vers sa seconde position projetée, ladite patte flexible (110) venant s'encliqueter sous ledit épaulement axial (108) lorsque ledit manchon actionneur (10) atteint sa seconde position projetée, après utilisation, verrouillant ainsi ledit manchon actionneur (10) par rapport audit corps (1).

9. Autoinjecteur selon la revendication 8, dans lequel ladite zone de réception finale (106) est décalée axialement par rapport à ladite zone initiale (102).

10. Autoinjecteur selon la revendication 8 ou 9, dans lequel ladite rainure finale (107) est inclinée et comporte ladite paroi axiale déformable (1020).

11. Autoinjecteur selon l'une quelconque des revendications 8 à 10, dans lequel ledit encliquetage de ladite patte flexible (110) dans ladite zone de réception finale (106) génère un son, tel qu'un clic.

12. Autoinjecteur selon l'une quelconque des revendications précédentes, dans lequel ladite déformation élastique de ladite paroi axiale déformable (1020) génère un son, tel qu'un clic.

13. Autoinjecteur selon l'une quelconque des revendications précédentes, dans lequel ladite tête (112) comporte une paroi d'extrémité frontale (1120) au moins partiellement inclinée adaptée à coopérer avec une extrémité inclinée de ladite paroi axiale déformable (1020) pour guider ladite tête (112) dans ladite rainure finale (107) lorsque ledit manchon actionneur (10) revient de sa position d'actionnement vers sa seconde position projetée.

## Patentansprüche

1. Autoinjektor, aufweisend einen Körper (1), der dazu geeignet ist, einen Behälter aufzunehmen, wobei der Behälter ein fluides Produkt enthält und einen Kolben und eine Nadel, wie eine vorgefüllte Spritze, aufweist, wobei der Autoinjektor eine Betätigungsmuffe (10) aufweist, die ein Kontaktende (11) aufweist, das dazu gedacht ist, mit dem Körper des Benutzers in Kontakt zu kommen, wobei die Betätigungsmuffe (10) in Bezug auf den Körper (1) zwischen vorstehenden Positionen, in denen die Betätigungsmuffe (10) zumindest teilweise aus dem Körper (1) vorsteht, und einer Betätigungsposition, in der die Betätigungsmuffe (10) zum Inneren des Körpers (1) axial verschoben ist, verschiebbar ist, wobei sich die Betätigungsmuffe (10) vor der Betätigung des Autoinjektors in einer ersten vorstehenden Position und nach der Betätigung des Autoinjektors in einer zweiten vorstehenden Position befindet, wobei eines von der Betätigungsmuffe (10) und dem Körper (1) eine flexible Lasche (110) aufweist, die dazu geeignet ist, sich seitlich in Bezug auf die Betätigungsmuffe (10) und/oder in Bezug auf den Körper (1) zu verformen, wenn die Betätigungsmuffe (10) von ihrer ersten vorstehenden Position in ihre Betätigungsposition und dann von ihrer Betätigungsposition zurück in ihre zweite vorstehende Position verschoben wird, wobei das andere von der Betätigungsmuffe (10) und dem Körper (1) eine Anfangszone (102), die in der ersten vorstehenden Position mit der flexiblen Lasche (110) zusammenwirkt, eine Zwischenzone (105), die in der Betätigungsposition mit der flexiblen Lasche (110) zusammenwirkt, und eine aufnehmende Endzone (106) aufweist, die in der zweiten vorstehenden Position mit der flexiblen Lasche (110) zusammenwirkt, wobei die aufnehmende Endzone (106) zumindest seitlich zu der Anfangszone (102) versetzt ist, **dadurch gekennzeichnet, dass** eine axiale verformbare Wand (1020) dazu geeignet ist, sich elastisch zu verformen, um die flexible Lasche (110) von der Anfangszone (102) zur Zwischenzone (105) passieren zu lassen, wobei die axiale verformbare Wand (1020) in ihrer nicht verformten Position anschließend dazu geeignet ist, die flexible Lasche von der Zwischenzone (105) zur aufnehmenden Endzone (106) zu führen, wobei die flexible Lasche (110) einen Kopf (112) aufweist, der in der Anfangsnut und der Endnut (101, 107) zwischen der Anfangszone (102), der Zwischenzone (105) und der aufnehmenden Endzone (106) gleitet.

2. Autoinjektor nach Anspruch 1, wobei die flexible Lasche (110) seitlich verformt wird, wenn sich die Betätigungsmuffe (10) von ihrer ersten vorstehenden Position vor der Betätigung zu ihrer Betätigungsposition verschiebt, und/oder die flexible Lasche (110) seitlich verformt wird, wenn sich die Betätigungsmuffe (10) am Ende der Verwendung von ihrer Betätigungsposition zu ihrer zweiten vorstehenden Position verschiebt.

3. Autoinjektor nach Anspruch 2, wobei die Betätigungsmuffe (10) mindestens eine Anfangsnut (101) aufweist, die die Anfangszone (102) mit der Zwischenzone (105) verbindet, wobei die Anfangsnut (101) die axiale verformbare Wand (1020) aufweist.

4. Autoinjektor nach Anspruch 3, wobei die Anfangsnut (101) einen Absatz (1010) aufweist, der seitlich in die Anfangsnut (101) vorsteht.

5. Autoinjektor nach Anspruch 4, wobei der Absatz (1010) gegenüber der axialen verformbaren Wand (1020) angeordnet ist.

6. Autoinjektor nach Anspruch 4 oder 5, wobei der Absatz (1010) eine geneigte Wand (1015) aufweist, die in Richtung der Anfangszone (102) gedreht ist, wobei die geneigte Wand (1015) die flexible Lasche (110) seitlich derart verformt, dass die flexible Lasche die axiale verformbare Wand (1020) verformt.

7. Autoinjektor nach einem der Ansprüche 3 bis 6, wobei die Anfangsnut (101) im Wesentlichen axial ist und durch eine zweite geneigte Nut (103) und/oder eine dritte axiale Nut (104) mit der Zwischenzone (105) verbunden ist.

8. Autoinjektor nach einem der vorhergehenden Ansprüche, wobei die aufnehmende Endzone (106) mit der Zwischenzone (105) durch eine Endnut (107) verbunden ist, wobei ein axialer Absatz (108) zwischen der aufnehmenden Endzone (106) und der Endnut (107) vorgesehen ist, wobei die flexible Lasche (110) dafür geeignet ist, in der Endnut (107) zu gleiten, wenn die Betätigungsmuffe (10) von ihrer Betätigungsposition zu ihrer zweiten vorstehenden Position zurückkehrt, wobei die flexible Lasche (110) unter dem axialen Absatz (108) zum Einrasten kommt, wenn die Betätigungsmuffe (10) nach der Verwendung ihre zweite vorstehende Position erreicht und so die Betätigungsmuffe (10) in Bezug auf den Körper (1) verriegelt.

9. Autoinjektor nach Anspruch 8, wobei die aufnehmende Endzone (106) axial in Bezug auf die Anfangszone (102) versetzt ist.

10. Autoinjektor nach Anspruch 8 oder 9, wobei die Endnut (107) geneigt ist und die axiale verformbare Wand (1020) aufweist.

11. Autoinjektor nach einem der Ansprüche 8 bis 10, wobei das Einrasten der flexiblen Lasche (110) in der aufnehmenden Endzone (106) ein Geräusch, wie ein Klicken, erzeugt.

12. Autoinjektor nach einem der vorhergehenden Ansprüche, wobei das elastische Verformen der axialen verformbaren Wand (1020) ein Geräusch, wie ein Klicken, erzeugt.

13. Autoinjektor nach einem der vorhergehenden Ansprüche, wobei der Kopf (112) eine Stirnendwand (1120) aufweist, die zumindest teilweise geneigt ist, die dazu geeignet ist, mit einem geneigten Ende der axialen verformbaren Wand (1020) zusammenzuwirken, um den Kopf (112) in der Endnut (107) zu führen, wenn die Betätigungsmuffe (10) von ihrer Betätigungsposition zu ihrer zweiten vorstehenden Position zurückkehrt.

## Claims

1. An autoinjector comprising a body (1) that is adapted to receive a reservoir, said reservoir containing fluid and including a piston and a needle, such as a pre-filled syringe, said autoinjector further comprising an actuator sleeve (10) that includes a contact end (11) for coming into contact with the user's body, said actuator sleeve (10) being movable relative to said body (1) between projecting positions in which said actuator sleeve (10) projects out from said body (1) at least in part, and an actuated position in which said actuator sleeve (10) is moved axially into said body (1), said actuator sleeve (10) being in a first projecting position before actuation of the autoinjector, and in a second projecting position after actuation of the autoinjector; one of said actuator sleeve (10) and of said body (1) including a flexible tab (110) that is adapted to deform laterally relative to said actuator sleeve (10) and/or relative to said body (1) when said actuator sleeve (10) is moved from its first projecting position to its actuated position, then from its actuated position to return to its second projecting position, the other of said actuator sleeve (10) and of said body (1) including an initial zone (102) that co-operates with said flexible tab (110) in said first projecting position, an intermediate zone (105) that co-operates with said flexible tab (110) in said actuated position, and a final reception zone (106) that co-operates with said flexible tab (110) in said second projecting position, said final reception zone (106) being offset, at least laterally, from said initial zone (102), the autoinjector being **characterized in that** a deformable axial wall (1020) is adapted to deform elastically so as to allow said flexible tab (110) to pass from said initial zone (102) to said intermediate zone (105), said deformable axial wall (1020), in its non-deformed position, then being adapted to guide said (105) flexible tab from said intermediate zone (105) to said final reception zone (106). flexible tab (110) includes a head (112) that slides in said initial and final grooves (101, 107) between said initial (102), intermediate (105, and final-reception zones (106)

2. An autoinjector according to claim 1, wherein said flexible tab (110) is deformed laterally when said actuator sleeve (10) is moved from its first projecting position, before actuation, to its actuated position, and/or said flexible tab (110) is deformed laterally when said actuator sleeve (10) is moved from its actuated position to its second projecting position, at the end of use.

3. An autoinjector according to claim 2, wherein said actuator sleeve (10) includes at least one initial groove (101) that connects said initial zone (102) to said intermediate zone (105), said initial groove (101) including said deformable axial wall (1020).

4. An autoinjector according to claim 3, wherein said initial groove (101) includes a shoulder (1010) that projects laterally into said initial groove (101).

5. An autoinjector according to claim 4, wherein said shoulder (1010) is arranged facing said deformable axial wall (1020).

6. An autoinjector according to claim 4 or claim 5, wherein said shoulder (1010) includes a sloping wall (1015) that faces towards said initial zone (102), said sloping wall (1015) deforming said flexible tab (110) laterally, in such a manner that said flexible tab deforms said deformable axial wall (1020).

7. An autoinjector according to any one of claims 3 to 6, wherein said initial groove (101) is substantially axial and is connected to said intermediate zone (105) via a sloping second groove (103) and/or via an axial third groove (104).

8. An autoinjector according to any preceding claim, wherein said final reception zone (106) is connected to said intermediate zone (105) via a final groove (107), an axial shoulder (108) being provided between said final reception zone (106) and said final groove (107), said flexible tab (110) being adapted to side in said final groove (107) when said actuator sleeve (10) returns from its actuated position to its second projecting position, said flexible tab (110) becoming snap-fastened below said axial shoulder (108) when said actuator sleeve (10) reaches its second projecting position after use, thereby locking said actuator sleeve (10) relative to said body (1).

9. An autoinjector according to claim 8, wherein said final reception zone (106) is offset axially relative to said initial zone (102).

10. An autoinjector according to claim 8 or claim 9, wherein said final groove (107) slopes and includes said deformable axial wall (1020).

11. An autoinjector according to any one of claims 8 to 10, wherein said snap-fastening of said flexible tab (110) in said final reception zone (106) generates a sound, such as a click.

12. An autoinjector according to any preceding claim, wherein said elastic deformation of said deformable axial wall (1020) generates a sound, such as a click.

13. An autoinjector according to any preceding claim, wherein said head (112) includes a front end wall (1120) that slopes at least in part, and that is adapted to co-operate with a sloping end of said deformable axial wall (1020) so as to guide said head (112) into said final groove (107) when said actuator sleeve (10) returns from its actuated position to its second projecting position.
